# FASCICULE DE BREVET EUROPEEN

(11) **EP 0 719 570 B1**
(45) Date de publication et mention de la délivrance du brevet: **09.04.2003**
(21) Numéro de dépôt: 95402975.7
(22) Date de dépôt: 29.12.1995
(51) Int. Cl.: A61N 1/37

(54) **Dispositif implantable comprenant des moyens de protection contre les perturbations électromagnétiques**
Implantierbare Vorrichtung mit Schutzmitteln gegen elektromagnetische Störungen
Implantable device with protection means against electromagnetic disturbances

(30) Priorité: 30.12.1994 FR 9415911
(43) Date de publication de la demande: 03.07.1996
(73) Titulaire: ELA MEDICAL (Société anonyme), F-92541 Montrouge (FR)
(72) Inventeur: Pons, Pascal, F-38320 Crolles (FR); Dal Molin, Renzo, F-92320 Chatillon (FR)
(74) Mandataire: Dupuis-Latour, Dominique

(56) Documents cités:
- EP-A- 0 228 539
- US-A- 4 739 437

## Description

L'invention concerne la protection des dispositifs implantables actifs, notamment des stimulateurs ou défibrillateurs cardiaques.

À cet égard, bien que dans la suite de la description on fasse principalement référence au cas d'un stimulateur, l'invention est applicable à tout type de "dispositif médical implantable actif' tel que défini par la directive 90/385/CEE du 20 juin 1990 du Conseil des communautés européennes.

Une telle protection est notamment destinée à garantir un fonctionnement normal du dispositif implantable quelles que soient les conditions environnantes, notamment en présence d'interférences électromagnétiques provenant de l'extérieur.

En effet, du fait des diverses électrodes reliées au dispositif implantable, ce dernier peut capter toutes sortes de perturbations électromagnétiques provenant de sources diverses telles que moteurs électriques, téléviseurs, plaques à induction, radiotéléphones, portiques de détection de systèmes d'accès ou de systèmes de protection antivol, etc. Il en est également de même pour un certain nombre d'appareils médicaux utilisés au cours d'interventions telles que bistouris électriques, instruments de cautérisation à courant alternatif, défibrillateurs, etc.

Ces perturbations peuvent être de natures très variées : surtensions brèves ou prolongées, courants induits, signaux radioélectriques de caractéristiques spectrales très diverses (s'étendant typiquement de 1 kHz à plusieurs mégahertz), etc., ce qui rend difficile la réalisation d'une protection du stimulateur qui soit complète et efficace en toutes circonstances.

Ces parasites extérieurs, souvent désignés sous le terme "EMI" (*ElectroMagnetic Interference*) et que l'on appellera ici "perturbations électromagnétiques", ou simplement "perturbations", ont pour premier effet de se superposer au signal cardiaque, en risquant de perturber le fonctionnement du stimulateur, qui doit donc les détecter et, si elles dépassent un seuil donné en dépit des protections ou circuits de suppression du stimulateur, ce dernier doit se placer en un mode "perturbation", où il fonctionnera de manière autonome tant que le niveau de la perturbation ne sera pas retombé au dessous d'un certain seuil.

Un autre effet des perturbations captées par le stimulateur est le risque de destruction dû à des tensions ou courants excessifs introduits dans les circuits du stimulateur ; il est donc absolument indispensable de limiter les tensions et les courants dans les sondes cardiaques en entrée de l'appareil.

Des normes très strictes ont d'ailleurs été définies en ce sens, et l'on pourra se référer à la norme CENELEC EN 50061, Amendement 1, "Sécurité des stimulateurs cardiaques implantables", qui définit les niveaux de protection minimaux requis ainsi qu'un certain de nombre de procédures de test pour vérifier la conformité des stimulateurs à la norme.

Jusqu'à présent, la protection à l'encontre des perturbations d'origine externe est réalisée au moyen de diodes Zener, sous forme de composants discrets rapportés sur le microcircuit du stimulateur et montés en entrée, à l'endroit du raccordement des diverses sondes. Ces diodes Zener assurent un écrêtage des surtensions susceptibles d'apparaître en entrée du stimulateur (écrêtage à la tension de Zener de la diode), le montage des diodes étant également conçu pour assurer une protection symétrique afin d'éviter en particulier tout effet de démodulation dans le cas de perturbations alternatives haute fréquence.

Une telle protection connue par diodes Zener ajoutées en entrée du stimulateur présente cependant l'inconvénient d'augmenter l'encombrement des circuits du stimulateur, du fait de la nécessité d'y rapporter des composants discrets supplémentaires, en nombre relativement élevé (chacune des bornes d'entrée/sortie du stimulateur nécessitant au moins deux diodes), ce qui va à l'encontre de la miniaturisation recherchée, tout particulièrement dans les appareils complexes tels que stimulateurs double et triple chambre, stimulateurs reliés à un capteur d'asservissement, défibrillateurs, etc.

En ce qui concerne leur efficacité, ces circuits de protection à diodes Zener assurent une protection satisfaisante à l'encontre des hautes tensions liées par exemple à des chocs de défibrillation.

En revanche, des tensions perturbatrices de plus faible amplitude peuvent être captées ou démodulées en cas d'interférences électromagnétiques et peuvent causer des problèmes avec les circuits basse tension du stimulateur. Les circuits classiques à diodes Zener sont inefficaces contre de telles perturbations.

Ce risque est tout particulièrement aggravé dans le cas où les circuits du stimulateur comportent des composants actifs intégrés fonctionnant avec des tensions nominales de commande (tension de grille de MOS, notamment) relativement basses, comme cela est par exemple décrit dans le US-A-4 739 437. Ce risque est d'ailleurs tel que jusqu'à présent ces composants à faible tension de commande n'ont pas été utilisés dans des dispositifs implantables, ce qui conduit à se priver des avantages des technologies de semiconducteurs les plus récentes, notamment leur très haute densité d'intégration et leur faible consommation ― caractéristiques pourtant particulièrement recherchées pour des dispositifs implantables autonomes.

L'un des buts de la présente invention est de proposer un dispositif implantable actif, notamment un stimulateur ou défibrillateur cardiaque, comportant des circuits de protection procurant une immunité à l'encontre de perturbations électromagnétiques présentant une très grande variété de caractéristiques électriques et spectrales, assurant ainsi en toutes circonstances un respect des normes de protection les plus sévères.

Un autre but de l'invention est de permettre la réalisation d'un tel dispositif selon une technologie mettant en oeuvre des tensions nominales de commande faibles, tout en procurant une protection conforme aux normes en vigueur.

Un autre but de l'invention est de contribuer à la miniaturisation du dispositif, en réduisant à un minimum le nombre de composants discrets rapportés des circuits de protection.

Un autre but de l'invention est de proposer des circuits de protection qui puissent être utilisables et adaptables à des technologies d'intégration diverses (par exemple technologie partiellement bipolaire et partiellement CMOS, ou technologie BiCMOS monolithique).

L'invention concerne un dispositif du type connu d'après le US-A-4 739 437, c'est-à-dire dont au moins tous les composants actifs des moyens de protection à l'encontre des perturbations électromagnétiques d'origine externe sont des composants intégrés de façon monolithique ainsi que le microcircuit comportant les étages d'entrée des signaux et de commutation des électrodes, et les étages de commutation comportent des commutateurs statiques à tension de commande supérieure à la tension de la pile d'alimentation du dispositif, le dispositif délivrant des signaux logiques de commande à tension inférieure à la tension de commande. Selon l'invention, les moyens de protection comprennent des moyens translateurs de tension pour élever, en valeur absolue, le niveau des signaux logiques de commande à une valeur compatible avec les tensions de commande des commutateurs statiques et supérieure ou égale au niveau nominal des tensions parasites résultant des perturbations électromagnétiques susceptibles d'apparaître sur les bornes des commutateurs statiques.

D'autres caractéristiques avantageuses sont présentées dans les sous-revendications.

D'autres caractéristiques et avantages de l'invention apparaîtront à la lecture de la description détaillée ci-dessous d'un exemple de réalisation, faite en référence aux dessins annexées.

La figure 1 est un schéma synoptique de l'un des microcircuits d'un stimulateur cardiaque, microcircuit qui comprend les circuits de commutation des électrodes, de protection et d'alimentation.

La figure 2 est un diagramme montrant les différentes tensions produites dans le circuit de la figure 1, et notamment les niveaux entre lesquels varient ces tensions.

La figure 3 est un schéma détaillé montrant la structure du circuit d'alimentation de la figure 1.

La figure 4 est un schéma agrandi d'une partie du circuit de la figure 1, montrant les organes de polarisation dynamique et de protection contre les surtensions.

La figure 5 est un schéma détaillé de la structure interne des organes de protection contre les surtensions des circuits des figures 1 et 4.

La configuration que l'on va décrire correspond à celle d'un stimulateur double chambre, mais cet exemple n'est bien entendu aucunement limitatif, l'invention pouvant s'appliquer aussi bien à des stimulateurs simple ou triple chambre, à des stimulateurs asservis ou non (donc comprenant une ou plusieurs électrodes supplémentaires de mesure d'un paramètre physiologique), à la partie de détection/stimulation d'un défibrillateur, ou même à des dispositifs implantables actifs autres que cardiaques.

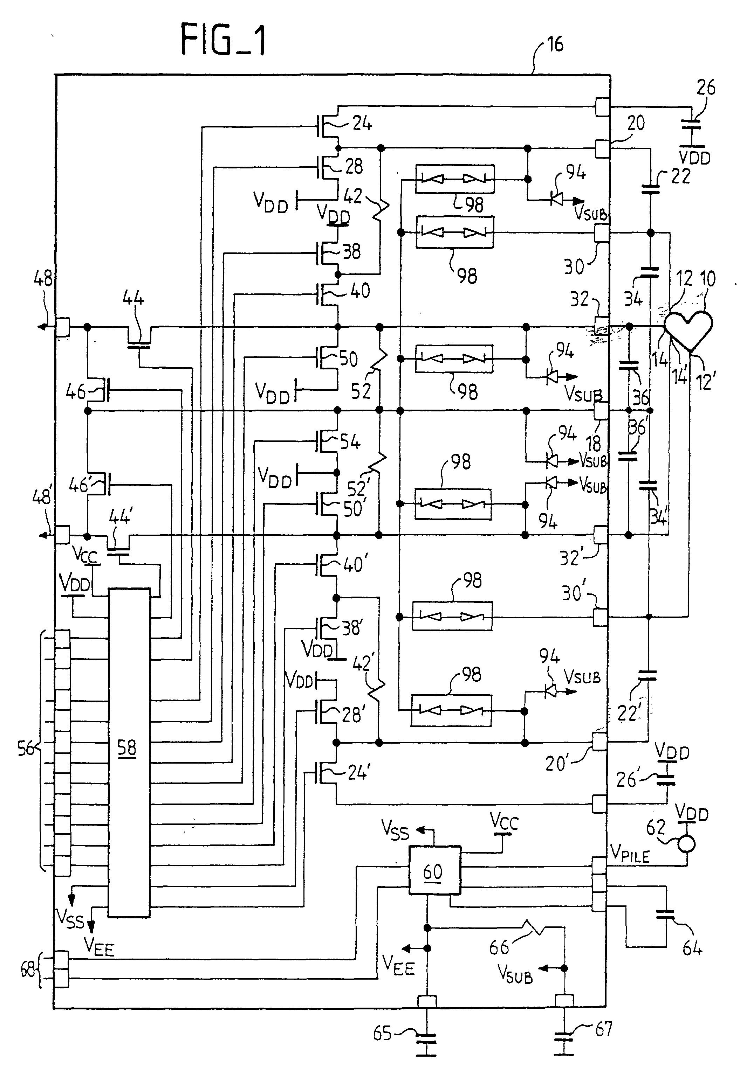

Sur la figure 1, la référence 10 désigne un coeur sur lequel ont été branchées diverses électrodes dont la configuration correspond à celle d'un stimulateur double chambre, à savoir des électrodes auriculaires distale 12 et proximale 14 et des électrodes ventriculaires distale 12' et proximale 14' (à cet égard, on notera que, dans l'exemple décrit, les circuits et éléments relatifs à la détection ou à la stimulation ventriculaire sont désignés par la même référence numérique, avec addition d'un "prime", que les circuits et éléments homologues semblables pour la stimulation ou la détection auriculaire).

Les différentes électrodes cardiaques sont reliées à un premier microcircuit 16 du stimulateur. Le boîtier du stimulateur est périodiquement relié à la masse du circuit, correspondant à la borne 18, notamment dans le cas d'une détection ou d'une stimulation unipolaires.

Les électrodes cardiaques et le boîtier ayant plusieurs états électriques (détection ou bien stimulation, mode monopolaire ou bien bipolaire), ceci implique la réalisation de commutations périodiques, commandées selon un séquencement particulier.

Plus précisément, l'électrode auriculaire 12 est reliée à une borne 20 via un condensateur de stimulation 22, la borne 20 pouvant être commutée par un commutateur 24 à un condensateur extérieur 26 dont l'autre borne est reliée au potentiel zéro (désigné V_{DD} sur le schéma, et correspondant à la borne positive de la pile), ou bien directement à V_{DD} par un autre commutateur 28.

Les électrodes auriculaires 12 et 14 sont également reliées à des bornes 30 et 32 destinées à permettre une détection bipolaire (par mesure de la tension différentielle) de l'activité cardiaque, ces bornes étant découplées par des condensateurs extérieurs respectifs 34 et 36 assurant le filtrage des perturbations haute fréquence (supérieures à 1 MHz).

Des commutateurs 38 et 40 mettent hors circuit (pendant la stimulation) ou en circuit (hors stimulation) une résistance d'entrée 42 pour l'évacuation de charges résiduelles subsistant après stimulation à l'interface coeur-électrode. Le commutateur 44 est passant en cas de détection bipolaire, et bloquant en cas de détection monopolaire, et le commutateur 46 a un fonctionnement inverse. Le signal détecté est ensuite dirigé via des lignes 48 vers des amplificateurs différentiels de détection (non représentés).

Le microcircuit 16 comporte également un commutateur 50 pour la mise en circuit de la masse auriculaire (cas d'une stimulation) ; en l'absence de commutation, l'électrode correspondante (borne 32) est reliée à la masse par l'intermédiaire d'une résistance 52 d'une dizaine de mégohms permettant de maintenir sur cette borne 32 un potentiel statique nul.

Le circuit de stimulation ventriculaire est le même que celui que l'on vient de décrire, les éléments 20' à 52' étant identiques aux éléments 20 à 52 que l'on vient de détailler.

Le circuit comporte enfin un commutateur 54 destiné à la mise en circuit sélective de la masse du boîtier (borne 18).

Ces divers commutateurs permettent d'assurer toutes les configurations de stimulation et de détection, aussi bien en mode bipolaire que monopolaire. Ce sont des commutateurs statiques à transistors tels que des MOS ou structures équivalentes.

Dans le cas d'un stimulateur asservi, ils peuvent être éventuellement complétés par un ou plusieurs commutateurs supplémentaires, par exemple, dans le cas d'un stimulateur asservi à l'activité respiratoire, pour assurer la décharge du condensateur d'injection.

D'autres commutations peuvent être également prévues, par exemple dans le cas d'un défibrillateur implantable.

Le rôle et le mode opératoire de ces différentes commutations sont en eux-mêmes bien connus, et ne seront pour cette raison pas décrits plus en détail.

Le microcircuit 16 de la figure 1, qui est un microcircuit dont tous les composants sont intégrés de manière monolithique, est associé à un second microcircuit (non représenté), également intégré de manière monolithique et comportant un circuit de commande logique, par exemple un circuit à microprocesseur ou une logique équivalente appropriée, en eux-mêmes bien connus. Ce circuit de commande logique délivre notamment des signaux logiques 56 de commande des commutateurs et des signaux d'horloge 68 utilisés par le microcircuit 16.

De façon caractéristique de l'invention, on commande les différents commutateurs à partir de signaux logiques non symétriques et de faible niveau (c'est-à-dire de niveau non supérieur à au niveau de tension de la pile), mais en produisant une tension de grille présentant un écart entre les tensions correspondant à l'état passant ou "ON" et l'état bloquant ou "OFF", écart ci-après désigné "excursion de tension", qui (1°) est très supérieur, en valeur absolue, aux valeurs des signaux logiques et qui (2°) est essentiellement symétrique par rapport au potentiel zéro, c'est-à-dire le potentiel de repos de toutes les entrées et sorties du stimulateur (potentiel V_{DD} = 0 V).

Les interférences éventuelles se superposant à ce potentiel de repos, une large excursion de tension permet de s'affranchir des risques de commutations intempestives, et la plage de tension symétrique par rapport au potentiel de repos évite tout risque lié à une démodulation de signal dans le cas d'une perturbation alternative haute fréquence.

Dans l'exemple illustré, les commutateurs sont des transistors MOS de type N dont la grille est commandée par une tension présentant l'excursion de tension particulière indiquée ci-dessus et dont le substrat est relié à une tension d'alimentation fortement négative.

Pour pouvoir commander les grilles dans cette dynamique, les commutateurs sont associés à des translateurs de tension respectifs 58 interposés entre les signaux de commande non symétriques et de faible niveau appliqués en 56 et les grilles des commutateurs correspondants.

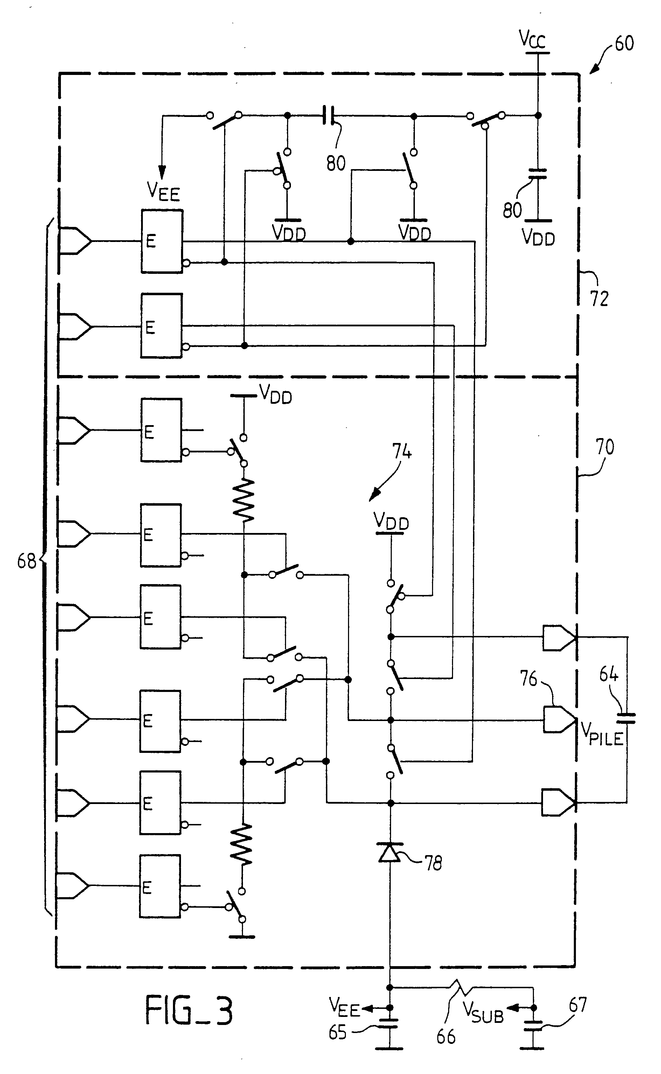

Ces translateurs de tension, dont on expliquera plus bas le rôle, reçoivent diverses tensions d'alimentation V_{DD}, V_{SS}, V_{CC} et V_{EE} produites par un bloc 60, dont la structure est représentée en détail figure 3.

Ce bloc 60, constitué d'éléments entièrement intégrés sous forme monolithique (comme tous les autres éléments du microcircuit 16), est relié à la borne négative d'une pile 62 dont l'autre borne constitue le potentiel zéro de référence (V_{DD}), les composants externes de l'alimentation étant un condensateur 64 et des condensateurs de découplage 65 et 67, dont les rôles seront expliqués plus loin.

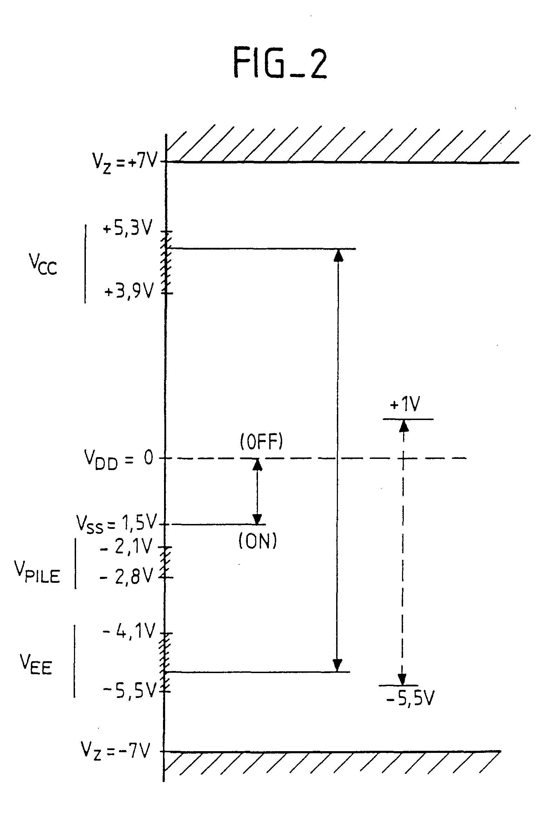

Les divers potentiels utilisés par le stimulateur de l'invention, et notamment les circuits que l'on vient de décrire, sont les suivants (on pourra notamment se reporter à la figure 2, où l'on a représenté sur un diagramme ces différents potentiels de manière à montrer leur importance relative et leur caractère symétrique ou non) :
- V_{DD} :: borne positive de la pile 62, correspond au potentiel zéro de référence.
- V_{PILE} :: borne négative de la pile, typiquement - 2,8 V (en début de vie de la pile) à - 2,1 V (en fin de vie de la pile).
- V_{SS} :: tension régulée négative -1,5 V ± 50 mV, utilisée spécifiquement pour les signaux logiques.
- V_{EE} :: tension négative, typiquement comprise entre - 5,5 V (début de vie) et - 4,1 V (fin de vie), produite par le bloc 60 à partir de V_{DD} et V_{PILE} (avec un condensateur de découplage 65).
- V_{CC} :: tension positive, typiquement + 5,3 V (début de vie) à + 3,9 V (fin de vie), produite par le bloc 60 à partir de V_{DD} et V_{EE} ; on notera que cette tension V_{CC} présente une valeur approximativement symétrique de la tension négative V_{EE} également produite par le bloc 60.
- V_{SUB} :: alimentation négative destinée à la polarisation des substrats ; cette tension est une tension dérivée de V_{EE} (avec un condensateur de découplage 67) avec interposition d'une résistance série de limitation (composant 66 sur la figure 1), typiquement une résistance intégrée de 300 kΩ ; V_{SUB} est donc comprise entre - 5,5 V et - 4,1 V. Plus précisément, la résistance 66 a pour objet, d'une part, de limiter le courant dans les sondes en cas de premier défaut et, d'autre part, de protéger VEE en présence d'un courant de substrat induit par une perturbation extérieure (par exemple le faible courant qui peut apparaître lors d'un écrêtage).

Les tensions sensiblement symétriques V_{CC} et V_{EE}/V_{SUB} sont produites de la manière suivante par le bloc 60 illustré figure 3.

Le bloc 60, qui reçoit des signaux d'horloge de commande 68 provenant du circuit logique du stimulateur, comporte essentiellement deux sous-ensembles, à savoir un sous-ensemble 70 produisant la tension négative V_{EE} (et donc V_{SUB} qui en est directement dérivée) à partir de V_{PILE}, et un sous-ensemble 72 produisant la tension positive V_{CC} à partir de la tension V_{EE} produite par le sous-ensemble 70.

Le sous-ensemble 70 est essentiellement constitué par un circuit doubleur de tension 74, de structure en elle-même connue et représentée sur la figure 3 (et que l'on n'explicitera pas plus pour cette raison). Ce circuit doubleur est formé d'une pluralité de translateurs séquencés de manière appropriée par les signaux logiques 68, de commutateurs associés et du condensateur externe 64. Il permet de transformer la tension de pile V_{PILE} comprise entre - 2,8 V et - 2,1 V en une tension V_{EE} (et donc une tension V_{SUB}) comprise entre - 5,5 V et - 4,1 V. La tension obtenue est délivrée via une diode 78 à faible tension de seuil, polarisée par un courant qui lui est appliquée depuis une source extérieure (non représentée) afin de limiter au maximum toute chute de tension au niveau de ce composant.

Le sous-ensemble 72, quant à lui, produit à partir de V_{EE} une tension positive V_{CC} comprise entre + 5,3 V et + 3,9 V, par exemple au moyen d'un circuit du type "pompe à charge" comprenant une pluralité de commutateurs séquencés par les signaux d'horloge 68 et des condensateurs intégrés associés 80, selon un schéma en lui-même connu et qui ne sera pas explicité plus en détail pour cette raison.

Un tel circuit du type pompe à charge est avantageux parce que, en technologie BiCMOS ou bipolaire à caissons isolés du substrat, il peut être réalisé entièrement intégré ; d'autres variantes de réalisation sont cependant possibles, par exemple, dans le cas d'une technologie CMOS simple, des circuits échantillonnés du type à rétablissement de niveau (*clamping*) ; dans cette dernière hypothèse, il sera nécessaire de prévoir autant de circuits que de commutateurs et de prévoir également un signal de rafraîchissement pour les commutateurs dont l'état passant doit être maintenu pendant la durée d'un cycle.

Grâce à la production de ces diverses tensions, il est possible, en appliquant des signaux logiques 56 (figure 1) compris entre V_{DD} et V_{SS} (0 V et - 1,5 V), de produire sur les grilles des commutateurs des tensions de commande dont l'excursion est comprise entre V_{EE} et V_{CC}, c'est-à-dire, typiquement, entre - 5,5 V et + 5,3 V (en début de vie de la pile ; entre - 4,1 V à 3,9 V en fin de vie de la pile), donc avec des tensions à la fois très supérieures - environ triplées en valeur absolue - et symétriques, assurant donc, et à double titre, une bien meilleure immunité aux perturbations extérieures susceptibles d'affecter la commande des commutateurs.

Les translateurs de tension 58, qui sont reliés aux divers potentiels V_{CC}, V_{DD}, V_{SS} et V_{EE} sont des circuits en eux-mêmes connus, interposés sur chaque ligne de transmission du signal logique de commande et assurant l'amplification et la symétrisation de la tension de commande V_{DD}/V_{SS} à V_{CC}/V_{EE}, donc l'amplification et la symétrisation de la dynamique de commande des commutateurs. Ces translateurs de tension peuvent être réalisés sous forme monolithique entièrement intégrée au microcircuit 16, donc avec possibilité d'une grande densité d'intégration et donc d'une miniaturisation extrême.

Grâce à cette configuration de circuit, on est assuré que la commande des divers commutateurs du circuit 16 sera à l'abri des perturbations électromagnétiques qui, autrement, auraient pu conduire dans certaines conditions à des commutations erronées et imprévisibles.

De plus, pour maintenir à tout instant, et en dépit de toutes les perturbations qui pourraient survenir, une tension V_{SUB} qui soit la tension la plus négative du circuit, on prévoit de doter le circuit d'une "alimentation dynamique" évitant que des perturbations fortement négatives ne viennent perturber les écarts relatifs entre les divers niveaux de tension produits par les translateurs de tension et appliqués aux divers commutateurs, avec des retentissements sur le fonctionnement d'ensemble du circuit.

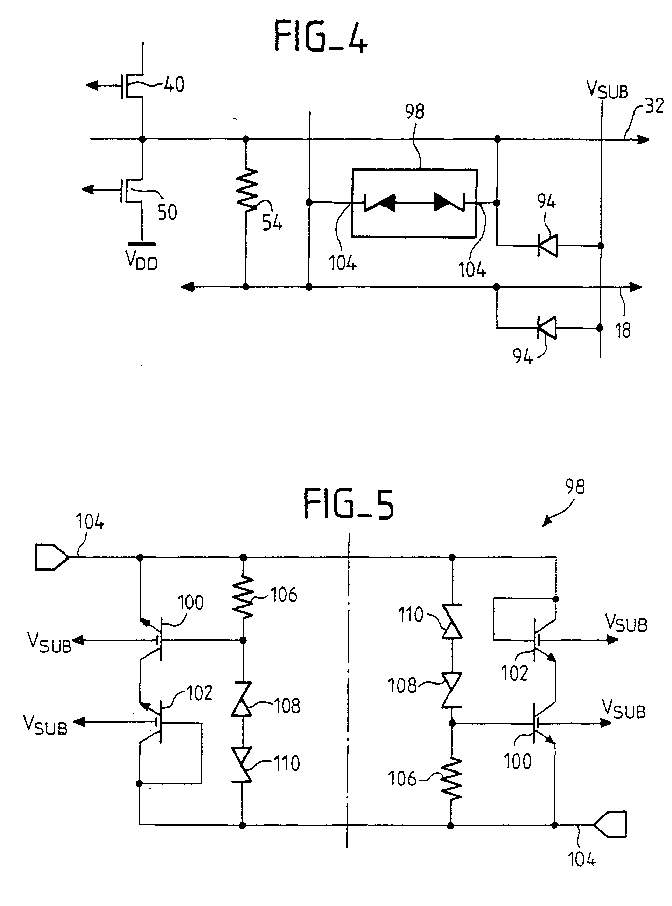

A cet effet, chacune des bornes 20, 20', 32, 32' et 18 est reliée, d'une part, à la cathode d'une diode 94 dont l'anode est reliée à V_{SUB} (les diodes 94 sont également visibles sur la vue partielle agrandie de la figure 4). En ce qui concerne la polarisation des masses auriculaire et ventriculaire, les résistances 52 et 52', qui relient les bornes 32 et 32' au boîtier (borne 18), permettent de s'affranchir de tout courant de fuite éventuel.

Ainsi, lorsque l'une quelconque des bornes 20, 20', 32, 32' et 18 est portée, du fait d'un parasite, à un potentiel plus négatif que V_{SUB}, les diodes entrent en conduction et permettent la suralimentation de l'ensemble des points reliés à V_{SUB} en évitant ainsi tout risque de commutation intempestive du fait de cette perturbation.

En ce qui concerne la protection contre les surtensions de forte valeur, la protection est habituellement réalisée, dans les stimulateurs connus, au moyen de diodes Zener montées en opposition entre chaque borne d'entrée et la masse. Ces diodes assurent un écrêtrage symétrique des tensions supérieures à la tension de Zener, typiquement des tensions supérieures en valeur absolue à ± 7 V (zones hachurées de la figure 2).

L'invention propose de remplacer ces composants, qui étaient jusqu'à présent des composants discrets rapportés sur le circuit, par des circuits de protection entièrement intégrés et monolithiques. Ces circuits selon l'invention sont des dipôles montés de la même façon que les diodes Zener en opposition de l'art antérieur, et susceptibles de conduire des niveaux de courant comparables à ces dernières (plusieurs centaines de milliampères), malgré leur structure entièrement intégrée et leur encombrement bien moindre.

Ces circuits d'écrêtage 98 sont en nombre égal à celui des bornes 20, 20', 30, 30', 32, 32' et sont montés chacun entre la borne respective et la masse du boîtier, correspondant à la borne 18.

Pour permettre leur intégration, ces circuits 98 ont chacun la structure illustrée figure 5, qui est une structure symétrique tête-bêche dont chacune des moitiés comporte deux transistors bipolaires 100, 102 montés en série, l'émetteur du transistor 100 étant relié à l'un des pôles 104 du circuit 98, le collecteur de ce transistor 100 étant relié à l'émetteur du transistor 102 et le collecteur du transistor 102 étant relié à l'autre pôle 104 du circuit 98. Entre la base et l'émetteur du transistor 100 est montée une résistance 106 et entre la base du transistor 100 et le collecteur du transistor 102 sont montées en opposition deux (ou plus) diodes Zener intégrées 108, 110 permettant de définir la tension d'écrêtage. Le dipôle ainsi constitué entre en conduction lorsque la différence de potentiel à ses bornes dépasse, en valeur absolue, 7 V. Il fonctionne alors en amplificateur de courant à conduction déclenchée par la tension, peut conduire entre ses bornes des courants jusqu'à 300 mA et peut absorber des pics de 3 A pendant 10 ms. Il est donc apte à supporter sans risque de destruction des perturbations même de très forte énergie.

## Revendications

1. Un dispositif implantable actif, notamment un stimulateur ou défibrillateur cardiaque, comportant des moyens de protection à l'encontre des perturbations électromagnétiques d'origine externe,
ces moyens de protection comportant des composants actifs et des composants passifs et au moins tous les composants actifs étant des composants intégrés de façon monolithique ainsi que le microcircuit (16) comportant les étages d'entrée des signaux et de commutation des électrodes,
ces étages de commutation comportant des commutateurs statiques (24, 24', 28, 28', 38, 38', 40, 40', 44, 44', 46, 46', 50, 50', 54) à tension de commande (V_{SUB}, V_{CC}) supérieure à la tension (V_{PILE}) de la pile d'alimentation du dispositif,
le dispositif délivrant des signaux logiques de commande à tension (V_{DD}, V_{SS}) inférieure à la tension de commande,
dispositif ***caractérisé en ce que*** les moyens de protection comprennent des moyens translateurs de tension (58) pour élever, en valeur absolue, le niveau des signaux logiques de commande à une valeur compatible avec les tensions de commande des commutateurs statiques et supérieure ou égale au niveau nominal des tensions parasites résultant des perturbations électromagnétiques susceptibles d'apparaître sur les bornes des commutateurs statiques.

2. Le dispositif implantable actif de la revendication 1, dans lequel les signaux logiques de commande étant des signaux de niveau inférieur au niveau nominal des tensions parasites, les moyens translateurs de tension délivrent des tensions de commande supérieures ou égales au niveau nominal des tensions parasites et symétriques, propres à éviter les phénomènes de démodulation de signal dans le cas de perturbations alternatives haute fréquence.

3. Le dispositif implantable actif de la revendication 1, dans lequel les niveaux des tensions de commande délivrées par les moyens translateurs sont produits par un circuit d'alimentation (60) élévateur de tension intégré monolithique.

4. Le dispositif implantable actif de la revendication 3, dans lequel le circuit d'alimentation élévateur de tension comporte au moins un circuit du type pompe à charge (72) et/ou un circuit du type doubleur de tension (70) et/ou un circuit du type échantillonné à rétablissement de niveau.

5. Le dispositif implantable actif de la revendication 3, dans lequel les substrats des commutateurs statiques sont polarisés à un potentiel correspondant au niveau de la tension de commande de même signe délivrée par le circuit d'alimentation (60) élévateur de tension.

6. Le dispositif implantable actif de la revendication 3, dans lequel les substrats des commutateurs statiques sont court-circuités à leur source.

7. Le dispositif implantable actif de la revendication 1, dans lequel les moyens de protection comportent en outre des moyens de suralimentation (94) comportant un composant relié, d'une part, à une borne d'entrée de signal et, d'autre part à une ligne d'alimentation délivrant un potentiel correspondant au niveau des tensions de commande produites par les moyens translateurs, ce composant étant susceptible d'entrer en conduction lorsque la tension sur la borne d'entrée correspondante dépasse, en valeur absolue, la tension sur ladite ligne d'alimentation, de manière à maintenir sur cette dernière une tension qui soit toujours, en valeur absolue, la plus élevée du circuit même en cas de perturbations de fort niveau.

8. Le dispositif implantable actif de la revendication 1, dans lequel lesdits étages d'entrée des signaux comportent des moyens écrêteurs (98) intégrés monolithiques reliés, d'une part, à une borne d'entrée de signal et, d'autre part, à un potentiel de masse du dispositif.

9. Le dispositif implantable actif de la revendication 8, dans lequel le circuit écrêteur comporte un circuit (100, 102, 106, 108, 110) tête-bêche amplificateur de courant à conduction déclenchée par la tension.

10. Le dispositif implantable actif de la revendication 9, dans lequel lesdits moyens écrêteurs comportent des circuits écrêteurs (98) comprenant chacun, sous forme intégrée et monolithique, un dipôle à structure symétrique tête-bêche dont chacune des moitiés comporte un premier (100) et un second (102) transistor bipolaire montés en série, l'émetteur du premier transistor étant relié à l'un des pôles (104) du circuit écrêteur, le collecteur de ce premier transistor étant relié à l'émetteur du second transistor et le collecteur du second transistor étant relié à l'autre pôle (104) du circuit écrêteur, une résistance (106) étant montée entre la base et l'émetteur du premier transistor, et au moins deux diodes Zener (108, 110) étant montées en opposition entre la base du premier transistor et le collecteur du second transistor.

11. Le dispositif implantable actif de la revendication 1, dans lequel les composants actifs des moyens de protection et le microcircuit sont intégrés sur le même substrat monolithique.

## Claims

1. An active implantable device, in particular a pacemaker or a defibrillator, the device having means for protecting it against electromagnetic disturbances of external origin,
these protection means comprising active components and passive components and at least all of the active components being monolithically integrated components as is the microcircuit (16) having the signal input stages and the electrode switching stages,
the switching stages comprising static switches (24, 24', 28, 28', 88, 88', 40, 40', 44, 44', 46, 46', 50, 50', 54) having a control voltage (V_{SUB}, V_{CC}) greater than the voltage (V_{PILE}) of the power supply battery of the device,
the device delivering logic control signals at a voltage (V_{DD}, V_{SS}) lower than the control voltage,
which device is **characterized in that** the protection means further comprise voltage shifting means (58) for raising the absolute value of the level of the control logic signals to a value that is compatible with the control voltages of the static switches and greater than or equal to the nominal level of interfering voltages that result from the electromagnetic disturbances that might appear on the terminals of the static switches.

2. The active implantable device of claim 1, in which the control logic signals being signals of level lower than the nominal level of the interfering voltages, the voltage shifting means deliver symmetrical control voltages greater than or equal to the nominal level of the interfering voltages and suitable for avoiding signal demodulation phenomena in the event of high frequency AC disturbances.

3. The active implantable device of claim 1, in which the control voltage levels delivered by the voltage shifting means are produced by an integrated monolithic voltage-raising power supply circuit (60).

4. The active implantable device of claim 3, in which the voltage-raising power supply circuit comprises at least one charge pump type circuit (72) and/or a voltage-doubling type circuit (70) and/or a circuit of the sampled type with level restoration.

5. The active implantable device of claim 3, in which the substrates of the static switches are biased to a potential corresponding to the same-sign control voltage level delivered by the voltage-raising power supply circuit (60).

6. The active implantable device of claim 3, in which the substrates of the static switches are short circuited at their source.

7. The active implantable device of claim 1, in which the protection means further comprise booster means (94) comprising a component connected firstly to a signal input terminal and secondly to a power supply line delivering a potential corresponding to the control voltage level produced by the voltage-shifting means, said component being suitable for entering into conduction when the voltage on the corresponding input terminal is of an absolute value that exceeds the voltage on said power supply line so as to maintain said line at a voltage whose absolute value is always the highest voltage of the circuit, even in the event of high level disturbances.

8. The active implantable device of claim 1, in which said signal input stages include integrated monolithic clipping means (98) connected firstly to a signal input terminal and secondly to a ground potential of the device.

9. The active implantable device of claim 8, in which the clipping circuit comprises a head-to-tail current amplifier circuit (100, 102, 106, 108, 110) whose conduction is triggered by voltage.

10. The active implantable device of claim 9, in which said clipping means comprise clipping circuits (98) each comprising in integrated and monolithic form a dipole of symmetrical head-to-tail structure in which each of the halves has a first and a second bipolar transistor (100, 102) connected in series, the emitter of the first transistor being connected to one of the poles (104) of the clipping circuit, the collector of said first transistor being connected to the emitter of the second transistor and the connector of the second transistor being connected to the other pole (104) of the clipping circuit, a resistor (106) being connected between the base and the emitter of the first transistor, and at least two zener diodes (108, 110) being mounted in opposition between the base of the first transistor and the collector of the second transistor.

11. The active implantable device of claim 1, in which the active components of the protection means and of the microcircuit are integrated on a common monolithic substrate.

## Patentansprüche

1. Eine aktive implantierbare Vorrichtung, insbesondere ein Herzschrittmacher oder ein Herzdefibrillator, aufweisend Schutzmittel gegen elektromagnetische Störungen externen Ursprungs,
wobei diese Schutzmittel aktive Komponenten und passive Komponenten aufweisen, und wobei zumindest alle aktiven Komponenten integrierte Komponenten sind in monolithischer Art, wie auch die Mirkoschaltung (16), welche die Eingangsstufen von Signalen und die Kommutationsstufen von Elektroden aufweist,
wobei diese Kommutationsstufen statische Schalter aufweisen (24, 24', 28, 28', 38, 38', 40, 40', 44, 44', 46, 46', 50, 50', 54) mit einer Steuerungsspannung (V_{SUB}, V_{CC}), die größer ist als die Spannung (V_{PILE}) der Versorgungsbatterie der Vorrichtung,
wobei die Vorrichtung logische Steuerungssignale mit einer Spannung (V_{DD}, V_{SS}) liefert, die niedriger ist als die Steuerungsspannung,
wobei die Vorrichtung **dadurch gekennzeichnet ist, dass** die Schutzmittel Spannungstranslatorenmittel (58) aufweisen, um im Absolutwert den Pegel der logischen Steuerungssignale auf einen Wert zu erhöhen, der kompatibel ist mit den Steuerungsspannungen der statischen Schalter und höher ist oder gleich dem nominalen Pegel parasitärer Spannungen, die aus elektromagnetischen Störungen resultieren, die in der Lage sind, auf den Anschlussklemmen der statischen Schalter aufzutreten.

2. Aktive implantierbare Vorrichtung nach Anspruch 1, bei welcher die logischen Steuerungssignale Signale mit einem Pegel sind, der niedriger ist als der nominale Pegel parasitärer Spannungen, wobei die Spannungstranslatorenmittel Steuerungsspannungen liefern, die größer oder gleich sind als der nominale Pegel der parasitären und symmetrischen Spannungen, geeignet um die Phänomene einer Signaldemodulation in dem Fall von Wechselstörungen hoher Frequenz zu vermeiden.

3. Aktive implantierbare Vorrichtung nach Anspruch 1, bei welcher die Pegel der Steuerungsspannungen, die durch die Translatorenmittel geliefert werden, erzeugt werden durch eine integrierte monolithische Spannungsheberversorgungsschaltung (60).

4. Aktive implantierbare Vorrichtung nach Anspruch 3, bei welcher die Spannungsheberversorgungsschaltung zumindest eine Schaltung des Typs Ladungspumpe (72) und/oder eine Schaltung des Typs Spannungsverdoppler (70) und/oder eine Schaltung des Abtasttyps mit Pegelwiederherstellung aufweist.

5. Aktive implantierbare Vorrichtung nach Anspruch 3, bei welcher die Substrate der statischen Schalter auf ein Potential vorgespannt sind, welches dem Pegel der Steuerungsspannung desselben Vorzeichens entspricht, der durch die Spannungsheberversorgungsschaltung (60) geliefert wird.

6. Aktive implantierbare Vorrichtung nach Anspruch 3, bei welcher die Substrate der statischen Schalter an ihrer Quelle kurzgeschlossen sind.

7. Aktive implantierbare Vorrichtung nach Anspruch 1, bei welcher die Schutzmittel unter anderem Aufladungsmittel (94) aufweisen, welche ein Bauteil aufweisen, das einerseits mit einer Signalseingangsanschlussklemme verbunden ist, andererseits mit einer Versorgungsleitung, welche ein Potential liefert, entsprechend den Spannungssteuerungspegeln, die durch die Translatorenmittel erzeugt werden, wobei dieses Bauteil geeignet ist, dann zu leiten, wenn die Spannung an der entsprechenden Eingangsanschlussklemme im Absolutwert die Spannung über diese Versorgungsleitung überschreitet, in der Weise, um auf dieser letzteren eine Spannung aufrechtzuerhalten, die im Absolutwert immer die höchste der Schaltung ist, selbst im Fall von Störungen hohen Niveaus.

8. Aktive implantierbare Vorrichtung nach Anspruch 1, bei welcher die Eingangsstufen der Signale monolithische integrierte Begrenzermittel (98) aufweisen, die einerseits mit einer Signaleingangsanschlussklemme verbunden sind und andererseits mit einem Massepotential der Vorrichtung.

9. Aktive implantierbare Vorrichtung nach Anspruch 8, bei welcher die Begrenzerschaltung eine Stromverstärker-Kopf-bei-Fuß-Schaltung (100, 102, 106, 108, 110) mit durch die Spannung ausgelöster Leitung aufweist.

10. Aktive implantierbare Vorrichtung nach Anspruch 9, bei welcher die Begrenzermittel Begrenzerschaltungen (98) aufweisen, die jeweils in integrierter und monolithischer Form einen Dipol mit symmetrischer Kopfbei-Fuß Struktur aufweisen, wovon jede der Hälften einen ersten (100) und einen zweiten (102) bipolaren Transistor aufweisen, die in Serie montiert sind, wobei der Emitter des ersten Transistors verbunden ist mit einem der Pole (104) der Begrenzerschaltung, der Kollektor dieses ersten Transistors verbunden ist mit dem Emitter des zweiten Transistors und der Kollektor des zweiten Transistors verbunden ist mit dem anderen Pol (104) der Begrenzerschaltung, wobei ein Widerstand (106) zwischen der Basis und dem Emitter des ersten Transistors montiert ist und zumindest zwei Zener-Dioden (108, 110) entgegengesetzt zwischen der Basis des ersten Transistors und dem Kollektor des zweiten Transistors montiert sind.

11. Aktive implantierbare Vorrichtung nach Anspruch 1, bei welcher die aktiven Komponenten der Schutzmittel und die Mikroschaltung auf demselben monolithischen Substrat integriert sind.
